# EUROPÄISCHE PATENTSCHRIFT

(11) **EP 0 923 704 B1**
(45) Veröffentlichungstag und Bekanntmachung des Hinweises auf die Patenterteilung: **23.10.2002**
(21) Anmeldenummer: 97938787.5
(22) Anmeldetag: 19.08.1997
(51) Int. Cl.: G01B 11/24, A61C 19/04

(54) **VERFAHREN UND VORRICHTUNG ZUR DURCHFÜHRUNG VON OPTISCHEN AUFNAHMEN**
OPTICAL IMAGING METHOD AND DEVICE
PROCEDE ET DISPOSITIF D'OBTENTION D'IMAGES PAR MOYENS OPTIQUES

(30) Priorität: 02.09.1996 DE 19636354
(43) Veröffentlichungstag der Anmeldung: 23.06.1999
(73) Patentinhaber: OraMetrix GmbH, 10179 Berlin (DE)
(72) Erfinder: RUBBERT, Rüdger, D-12101 Berlin (DE)
(74) Vertreter: Weber, Dieter, Dr.
(86) Internationale Anmeldenummer: DE9701796
(87) Internationale Veröffentlichungsnummer: WO98010243

(56) Entgegenhaltungen:
- EP-A- 0 250 993
- US-A- 5 309 243
- US-A- 5 372 502

## Beschreibung

Die Erfindung betrifft ein Verfahren zur Durchführung von optischen Aufnahmen, bei dem mindestens zwei Einzelaufnahmen nacheinander erfolgen und für die Einzelaufnahmen die für die Bildwandlung wirksame Menge der Strahlenergie unterschiedlich gestellt wird. Ferner betrifft die Erfindung eine Vorrichtung für ein solches Verfahren.

Zum Zweck der Darstellung, Dokumentation oder Vermessung bietet der Einsatz von Verfahren, die auf optischer Grundlage arbeiten, eine Vielzahl von Vorteilen. Eine Vermessung kann schnell und berührungslos erfolgen. Zum Stand der Technik gehören elektronische Bildwandler, beispielsweise CCD-Arrays, deren Ausgangssignale unmittelbar nach einer Digitalisierung gespeichert oder ausgewertet werden können.

Bekannt sind Verfahren und Vorrichtungen zur Darstellung und zur optischen dreidimensionalen Vermessung von räumlichen Oberflächen. Sie basieren auf Triangulationsverfahren, bei denen unter einem bestimmten Winkel Punkt-, Linien- oder beliebige andere Muster auf die betrachtete Oberfläche projiziert werden und die projizierten Muster unter einem anderen Blickwinkel mit einer Optik und einem Bildwandler aufgenommen werden. Die bekannte Geometrie zwischen Projektionsrichtung und Aufnahmerichtung erlaubt die dreidimensionale Berechnung von Stützpunkten der Oberfläche.

Im Bereich der Zahnmedizin ist unter dem Markennamen CEREC bespielsweise ein System zur Herstellung von Keramikinlays bekannt, bei dem zum Vermessen einer Kavität eines Zahns eine optische 3D-Meßvorrichtung verwendet wird.

Für kieferorthopädische Anwendungen wird in der deutschen Patentanmeldung P 42 18 219 eine Intraoral-Stereo-Kamera beschrieben. Diese wird als Teil eines Medizingeräts, das unter dem Markennamen bending art system vertrieben wird, verwendet. Die räumlichen Informationen, die mit Hilfe dieser Kamera aus stereoskopischen Teilaufnahmen des Zahnbogens gewonnen werden, werden nachträglich miteinander kombiniert und zu einer räumlichen Gesamtinformation des Zahnbogens zusammengefügt.

Bei der Anwendung von optischen Verfahren zu Vermessungs- und Dokumentationszwecken treten jedoch immer dann Probleme auf, wenn die zu erfassende Oberfläche ungünstige Reflexionseigenschaften aufweist. Bei Aufnahmen von Zähnen stellt sich beispielsweise gegebenenfalls die Aufgabe, sowohl dunkle und matte Amalgamfüllungen als auch stark reflektierende Goldfüllungen gleicherrnaßen mit ausreichendem Informationsgehalt zu erfassen. Zudem ist der Dynamikbereich von preiswerten CCD-Arrays eingeschränkt.

In der US-Patentschrift US 5,309,243 wird ein Verfahren beschrieben, bei dem mittels niedrigauflösenden elektronischen Bauteilen analoge Bildsignale digitalisiert und verarbeitet werden. Dieses Verfahren, das auf feste und bewegliche Videokameras sowie Filmscanner anwendbar sein soll, beruht im wesentlichen darauf, daß die Belichtung vieler einzelner Bildpunkte eines Bildes mit verschiedenen Belichtungsstufen analog gemessen wird. Die Veränderung der Belichtung wird bei diesem Verfahren durch die Weite der Verschlußöffnung bzw. die Belichtungszeit erreicht.

Aufgabe der Erfindung ist es daher, das Verfahren und die Vorrichtung zur Durchführung von optischen Aufnahmen der eingangs beschriebenen Art zu schaffen, die die Erfassung eines größeren Umfangs von Bildinformationen über das betrachtete Objekt ermöglicht, als aufgrund der bauartbedingten Grenzen des mindestens einen verwendeten Bildwandlers in einem Einzelbild zur Verfügung stehen.

Die Aufgabe wird erfindungsgemäß bei dem Verfahren dadurch gelöst, daß
a) ein Aufnahmevorgang unter Verwendung mindestens eines flächigen elektronischen Bildwandlers durchgeführt wird, in dessen Verlauf mindestens zwei Einzelbilder gewonnen werden und
b) für die Einzelaufnahmen die für die Bildwandlung wirksame Menge der Strahlenergie unterschiedlich gestellt wird.

Unter "Menge der Strahlenergie" ist **im Sinne dieser Erfindung** das Integral des Produkts aus Wirkungsdauer und Intensität einer Strahlung zu verstehen.

Unter einer Beeinflussung der "für die Bildwandlung wirksamen Menge der Strahlenergie" ist **im Sinne dieser Erfindung** ausschließlich eine gezielte Beeinflussung der für die Bildwandlung wirksamen Menge der Strahlenergie seitens der Einrichtungen der Aufnahmevorrichtung selbst zu verstehen. Daß zusätzlich das zu erfassende Objekt selbst diese für die Bildwandlung wirksame Menge der Strahlenergie wesentlich - auch im Verlauf des Aufnahmevorgangs unterschiedlich - beeinflußt, ist selbstverständlich.

Die für die Einzelaufnahmen wirksame Menge der Strahlenergie kann erfindungsgemäß vorteilhaft in unterschiedlicher Art und Weise verändert werden, indem
a) mindestens eine Strahlquelle in ihrer Intensität und/oder Wirkungsdauer für die Aufnahme der Einzelbilder unterschiedlich gestellt wird;
b) in mindestens einem Strahlengang zwischen einer Strahlquelle und dem zu erfassenden Objekt optische Mittel derart gestellt werden, daß die Intensität und/oder die Wirkungsdauer der Strahlenenergie zur Beleuchtung bzw. Durchleuchtung des Objekts für die Aufnahme der Einzelbilder verändert wird;
c) in mindestens einem Strahlengang zwischen dem Objekt und einem Bildwandler optische Mittel derart gestellt werden, daß die Intensität und/oder die Wirkungsdauer der vom Objekt reflektierten bzw. nicht absorbierten Strahlenenergie für die Aufnahme der Einzelbilder verändert wird;
d) sogenannte Shutter-Einrichtungen des Bildwandlers derart angesteuert werden, daß die wirksame Zeitdauer der optoelektronischen Wandlung der für die Bildwandlung in den Bildwandler eingebrachten Strahlenenergie für die Einzelaufnahmen unterschiedlich gestellt wird.

Eine Strahlquelle **im Sinne dieser Erfindung** ist jeder direkte Erzeuger von Strahlenergie. Darunter fallen unter anderem Einrichtungen zur Erzeugung von Röntgenstrahlen, sichtbarem, infrarotem und ultraviolettem Licht.

Optische Mittel im Strahlengang zwischen Strahlquelle und Objekt bzw. zwischen Objekt und Bildwandler, die es erlauben, die Intensität und/oder die Wirkungsdauer der Strahlenergie zur Beleuchtung/Durchleuchtung des Objektes bzw. zur Bestrahlung des Bildwandlers für die Aufnahme der Einzelbilder unterschiedlich zu stellen, sind **im Sinne dieser Erfindung** optische Mittel, die die Intensität der Strahlenergie - bis auf Randeffekte - für die gesamte Fläche gleichmäßig verändern, also beispielsweise Blenden, mechanische und LCD-Shutter. Optische Mittel, die in veränderlicher Art und Weise Muster auf die Oberfläche des Objektes projizieren, fallen **nicht** unter diese Definition. Ein solches Verfahren, das sich ausschließlich auf eine frei programmierbare Projektionseinheit beschränkt, ist in der US 5,372,502 beschrieben.

Es sind Aufnahmeeinheiten bekannt, in denen Blenden oder auch Shutter-Einrichtungen bei Bildwandlern stellbar ausgeführt werden. Stand der Technik ist es, diese Einrichtungen in automatischen Regelkreisen zur Steuerung der Belichtung in der Weise anzusteuem, daß die Belichtung der Einzelaufnahmen optimiert wird. Der Terminus "für die Einzelaufnahmen unterschiedlich gestellt" bedeutet **im Sinne** **dieser Erfindung**, daß die oben genannten Mittel für aufeinanderfolgende Aufnahmen unterschiedlich gestellt werden, um gezielt im Verlauf des Aufnahmevorgangs den Gehalt der Bildinformationen aufeinanderfolgender Einzelaufnahmen zu variieren.

In einer besonders vorteilhaften Ausgestaltung der Erfindung wird mindestens eine Strahlquelle für die Einzelaufnahmen in seiner Intensität unterschiedlich gestellt.

Es ist erfindungsgemäß vorteilhaft, zusätzlich oder alternativ zur stellbaren Strahlquelle eine stellbare Blende ggf. mit geeigneter Optik in den optischen Strahlengang einzubringen, die hinsichtlich Öffnungszeit und/oder Brennweite für die Einzelaufnahmen unterschiedlich gestellt werden kann. Diese Blende kann gleichermaßen vorteilhaft im optischen Strahlengang zwischen einer Strahlquelle und dem Objekt oder zwischen dem Objekt und dem Bildwandler angeordnet sein.

Alternativ kann erfindungsgemäß vorteilhaft anstelle der Blende beispielsweise ein mechanischer Shutter oder eine LCD-Einrichtung angeordnet werden, die für die Einzelbilder unterschiedlich gestellt werden können. Die LCD-Einrichtung kann erfindungsgemäß vorteilhaft flächig durchlässig, teilweise durchlässig oder auch undurchlässig gestellt werden.

Zum Stand'der Technik gehören sogenannte elektronische Shutter-Einrichtungen von CCD-Bildwandlem, mit deren Hilfe die Taktzeit für das Shiften der Ladungen aus den für die Belichtung vorgesehenen Bereichen des CCD-Arrays in optisch abgedeckte Bereiche derart gewählt werden kann, daß die Integrationszeit des Bildwandlers und damit die Helligkeit des aufgenommen Bildes verändert werden kann. Entsprechend ist es erfindungsgemäß vorteilhaft, mittels geeigneter Ansteuerung dieser Einrichtung, die für die Bildwandlung wirksame Menge der Strahlenergie für die Einzelbilder unterschiedlich zu stellen.

Eine besonders vorteilhafte Ausgestaltung der Erfindung sieht vor, daß die gewonnenen Einzelbildinformationen digitalisiert werden und die digitalisierten Daten einer Einrichtung für die elektronische Datenverarbeitung zur Verfügung gestellt werden.

Einige handelsübliche CCD-Arrays werden beispielsweise mit einer Bildwechselfrequenz von 50Hz betrieben, es werden also fünfzig Aufnahmen pro Sekunde vorgenommen. Es ist Stand der Technik, mittels elektronischer Datenverarbeitung die erhaltenen Bilder zu speichern und zu verarbeiten. Beispielsweise sind sogenannte "Frame-Grabber" preisgünstig als Steckkarten für Arbeitsplatzcomputer erhältlich, die ein Videosignal direkt digitalisieren, in ein geeignetes Datenformat umwandeln und dem Computer als Daten in dessen Hauptspeicher zur Verfügung stellen. Diese Verarbeitung kann in Echtzeit erfolgen.

Wenn nun in erfindungsgemäß vorteilhafter Art und Weise unter Zugrundelegung der vorstehend beschriebenen Aufnahmeeinheit eine Beleuchtungsquelle in wiederholter Folge für eine 1/50stel Sekunde mit geringer Helligkeit und anschließend für eine weitere 1/50stel Sekunde mit hoher Helligkeit synchron zur Bildwechselfrequenz des CCD-Arrays die zu erfassende Oberfläche beleuchtet, erhält man als Ergebnis abwechselnd Bilder mit einer geringen und einer starken Belichtung. Die gering belichteten Bilder werden die Teile der Oberfläche mit hohem Reflexionsgrad zufriedenstellend abbilden, während die Teile der Oberfläche mit geringem Reflexionsgrad nur sehr dunkel und ohne nennenswerten Informationsgehalt abgebildet werden. Umgekehrt werden bei den stark belichteten Bildern die schwach reflektierenden Oberflächenanteile ausreichend hell und mit hohem Informationsgehalt abgebildet, während die stark reflektierenden Oberflächenteile durchgängig extrem hell und damit überstrahlt erscheinen.

Es ist erfindungsgemäß besonders vorteilhaft, die genannten optischen Mittel zur Beeinflussung der für die Bildwandlung wirksamen Menge der Strahlenergie synchron zur Bildwechselfrequenz anzusteuern.

Es ist zusätzlich erfindungsgemäß besonders vorteilhaft, wenn die mindestens zwei einzelnen Bildern zugehörigen Daten teilweise oder vollständig mittels geeigneter Algorithmen in der Einrichtung für die elektronische Datenverarbeitung kombiniert werden.

Beispielsweise ist es erfindungsgemäß vorteilhaft möglich, die digitalisierten Daten zweier aufeinanderfolgender Bilder mittels entsprechender Software dergestalt zu kombinieren, daß aus dem oben beschriebenen niedrig belichteten Einzelbild diejenigen Bildbereiche mit hohem Reflexionsgrad und aus dem oben beschriebenen stark belichteten Einzelbild die Bildbereiche mit niedrigem Reflexionsgrad ausgewählt und in eine gemeinsame Helligkeitsskala transformiert werden. Als Ergebnis erhält man ein Bild, das alle Bildbereiche mit ausreichendem Kontrast darstellt.

Erfindungsgemäß vorteilhaft ist es, eine feinere Abstufung der Beleuchtungsstärke vorzusehen. Dabei können weitere Helligkeitsstufen eingesetzt werden. Das kombinierte Bild wird dann aus den Bildinformationen mehrerer Einzelaufnahmen zusammengesetzt.

Es ist erfindungsgemäß nicht erforderlich, ausschließlich direkt aufeinanderfolgende Bilder auszuwerten oder nur Einzelbilder zu kombinieren, die mit unterschiedlicher Strahlenergie aufgenommen wurden.

Erfindungsgemäß besonders vorteilhaft ist eine automatische Regelung der genannten optischen Mittel zur Beeinflussung der für die Bildwandlung wirksamen Menge der Strahlenergie, abhängig von der Auswertung der tatsächlichen Reflexionsverhältnisse in den vorher aufgenommenen Bildern der jeweiligen Oberfläche. Durch diesen Regelkreis wird erfindungsgemäß sichergestellt, daß für alle Bereiche der zu erfassenden Oberfläche die Beleuchtungsstärke jeweils optimiert wird.

Ebenfalls erfindungsgemäß vorteilhaft ist die Verwendung mehrerer Strahlquellen. Beispielsweise kann dann mittels einer Strahlquelle und geeigneter optischer Mittel ein Muster auf die zu vermessende Oberfläche projiziert werden. Wenn der Projektionswinkel unterschiedlich vom Aufnahmewinkel ist, können in bekannter Weise aus der Abbildung des Musters auf dem Bildwandler 3D-Informationen errechnet werden. Die andere Strahlquelle leuchtet dabei alternierend die zu vermessende Oberfläche vollständig aus. Dann können aus den Einzelbildern sowohl 3D-Informationen als auch Ansichten der erfaßten Oberfläche gewonnen werden. Der erfindungsgemäße Vorteil liegt darin, daß sich eine entsprechende Vorrichtung ohne mechanische Bewegung der optischen Mittel, bei einfacher Ausbildung des Strahlengangs und unter Verwendung marktüblicher Einzelteile aufbauen läßt.

Auf diese Art und Weise können erfindungsgemäß vorteilhaft optische Strahlengänge sowohl für die Beleuchtung bzw. Durchleuchtung des zu erfassenden Objekts als auch für die Abbildung des Objekts auf dem oder den Bildwandlern durch die Ansteuerung der hierfür in dieser Erfindung als geeignet benannten optischen Mittel (beispielsweise stellbare Strahlquellen oder Blenden ggf. mit geeigneten Optiken) wirksam bzw. unwirksam geschaltet werden.

Eine weitere vorteilhafte Ausgestaltung der Erfindung verwendet mindestens zwei Strahlquellen und mindestens zwei Bildwandler, wobei durch geeignete optische Einrichtungen, z. B. durch einen Strahlteiler erreicht wird, daß die jeweilige Bildinformation auf beide Bildwandler projiziert wird. Wenn einer der beiden Bildwandler nicht für sichtbares Licht, sondern für beispielsweise infrarotes oder ultraviolettes Licht ausgelegt ist und einer der beiden Strahlquellen ebensolches Licht liefert, kann durch alternierendes Ansteuern der einen oder anderen Strahlquelle und durch die entsprechende Auswahl des Bildsignals des korrespondierenden Bildwandlers erreicht werden, daß abwechselnd Bildinformationen aus dem sichtbaren Lichtbereich und Bildinformationen aus einem nicht sichtbaren Bereich akquiriert werden.

In diesem Zusammenhang ist es erfindungsgemäß vorteilhaft möglich, die Daten des Bildwandlers für sichtbares Licht auf einem Monitor anzuzeigen und die Daten des Bildwandlers für nicht sichtbares Licht durch Datenverarbeitung so zu bearbeiten, daß sie in aussagekräftiger Form ebenfalls am Monitor dargestellt werden können. Bei Verwendung von infrarotem Licht können die erhaltenen unterschiedlich starken Signale beispielsweise durch unterschiedliche Farben gekennzeichnet werden, so wie dies bei der Auswertung von Infrarotbildern zur Überprüfung der Wärmeabstrahlung von Gebäuden üblich ist. Der Benutzer kann für die Monitordarstellung dann wählen zwischen dem Farb- bzw. Kontrastbild und der Auswertung der nicht sichtbaren Strahlen.

Eine weitere vorteilhafte Ausgestaltung der Erfindung verwendet mindestens eine Strahlquelle mit extrem kurzer Leuchtdauer, vorzugsweise im Bereich von 0,001 bis 0,01 s, und großer Helligkeit. Die für die Belichtung des zu erfassenden Objekts abgestrahlte Energiemenge kann in diesem Fall durch die Strahldauer variiert werden. Zudem wird der Einfluß von Verwackelungseffekten bei unruhiger Handhabung der Aufnahmevorrichtung minimiert. Solche Strahlquellen sind beispielsweise bekannt als Stroboskoplicht, Blitzlampe, Blitzrohr oder Flash-LED und werden beispielsweise (ebenfalls extern getaktet) zum Einstellen des Zündzeitpunktes bei Ottomotoren mit mechanischem Unterbrecherkontakt verwendet.

Beim Aufnahmevorgang können "abgeschattete" Bereiche entstehen, wenn die zu erfassende Oberfläche bezüglich des Strahlengangs des projizierten Musters Hinterschneidungen aufweist. Hinterschneidungen in diesem Sinne heißen Ausbildungen der dreidimensionalen Kontur, die bezüglich der Betrachtungs- oder der Projektionsrichtung durch Teile des Objekts selbst verdeckt sind und insofern für eine Betrachtung oder Projektion nicht zugänglich sind. Für solche Fälle ist es erfindungsgemäß besonders vorteilhaft, mindestens eine weitere Strahlquelle vorzusehen, die alternierend zu den verwendeten anderen Strahlquellen mit Hilfe geeigneter optischer Mittel ein Muster aus einer weiteren Raumrichtung auf die Oberfläche projiziert. Damit können Informationen über diejenigen vorher abgeschatteten Bereiche der Oberfläche gewonnen werden, die unter dem neuen Projektionswinkel optisch zugänglich sind. Mittels entsprechender Software können die unter den unterschiedlichen Projektionswinkeln erhaltenen 3D-Informationen der zu erfassenden Oberfläche dergestalt kombiniert werden, daß sich die 3D-lnformationen gegenseitig ergänzen.

Sämtliche in dieser Erfindung beschriebenen Ausgestaltungen des Verfahrens und der Vorrichtung können erfindungsgemäß besonders vorteilhaft in vielfältiger Art und Weise miteinander kombiniert werden.

Beim Vermessen von schlecht zugänglichen Stellen ist die Baugröße desjenigen Teils der Vorrichtung, der sich im schlecht zugänglichen Bereich befindet, begrenzt. Ein Beispiel hierfür sind Aufnahmen in der Mundhöhle. Es ist aus diesem Grunde nicht ohne weiteres möglich, eine Aufnahme beispielsweise eines kompletten Kiefers anzufertigen. Bewegt man nun die erfindungsgemäße Aufnahmevorrichtung relativ zur vermessenen Oberfläche, erhält man in Folge unterschiedliche Bilder. Die sich an die Einzelaufnahmen anschließende Datenverarbeitung bietet dann die Möglichkeit, benachbarte Einzelbilder zusammenzufügen, sofern ein ausreichender Überdeckungsgrad der Einzelbilder gewährleistet ist.

Es ist bekannt, daß aus einer relativ zur betrachteten Oberfläche veränderten Position und Ausrichtung der Aufnahmevorrichtung eine mehr oder weniger starke Abweichung der Abbilder für ein und dieselben betrachteten Oberflächensegmente resultiert. Insofern ist es Stand der Technik, numerische Näherungsverfahren zur Kombination und Ergänzung von benachbarten Bildern bzw. Bildfolgen einzusetzen. Geeignete numerische Algorithmen erlauben es, die Verzeichnungsfehler der ebenen Abbildung der realen, meist räumlich ausgebildeten Oberfläche teilweise zu kompensieren. Dem kontinuierlichen Zusammenfügen ebener Bilder räumlicher Objekte sind jedoch Grenzen gesetzt. Die unterschiedlichen Methoden zur ebenen Darstellung der Erdoberfläche geben hier ein anschauliches Beispiel.

Es liegt deshalb in diesem Zusammenhang nahe, daß nicht nur die zweidimensionalen Abbilder zu einem Gesamtbild zusammengefügt werden, sondern ermittelte 3D-Information. Entsprechende sogenannte Matching-Algorithmen, die es erlauben, Teilinformationen über dreidimensionale Oberflächen anhand von 3D-Koordinaten übereinstimmender Oberflächensegmente zu einer Gesamtinformation zu kombinieren, sind bekannt.

Die Voraussetzung übereinstimmender Oberflächensegmente ist für den oben beschriebenen beispielhaften Einsatzzweck der Erfindung gegeben. Da die erfindungsgemäße Vorrichtung bei Verwendung eines Bildwandlers mit einer Bildwechselfrequenz von 50Hz alle 0,02 s Einzelaufnahmen anfertigt, kann davon ausgegangen werden, daß selbst dann, wenn die Vorrichtung beispielsweise von Hand für eine Aufnahme des Zahnbogens eines Patienten geführt wird, jedes Bild aus einer ähnlichen Position aufgenommen wird wie die Vorgängeraufnahme und daß damit ein hoher Überdeckungsgrad zwischen zwei aufeinanderfolgenden Aufnahmen gewährleistet ist, sowohl für die 2D-Informationen als auch für die gewonnenen 3D-Informationen. Eine typische Größe des innerhalb einer Aufnahme erfaßten Teils der zu vermessenden Oberfläche liegt bei etwa 10mm x 10mm, so daß sich selbst bei einer hoch angesetzten Bewegungsgeschwindigkeit einer handgeführten Vorrichtung von ca. 30mm/s ein Überdeckungsgrad von mehr als 90% ergibt. Auch wenn davon ausgegangen wird, daß von den weiter oben beschriebenen Möglichkeiten der alternierenden Belichtung und Auswertung Gebrauch gemacht und beispielsweise nur jedes vierte Bild tatsächlich für eine dreidimensionale Berechnung herangezogen wird, resultiert daraus immer noch ein Überdeckungsgrad von über 75%.

Bei einer solchen vorteilhaften Ausgestaltung der Erfindung wird durch Bewegen der Vorrichtung entlang der Zahnreihen mit jedem ausgewerteten Bild eine neue Information über die dreidimensionale Gestalt der zu vermessenden Oberfläche an die bereits vorhandene angefügt. Zudem kann durch numerische Kombination der auf die Einzelbilder bezogenen 3D-Daten hinsichtlich des sich jeweils überdeckenden Bereichs die resultierende Information optimiert werden. Sie wird dabei durch Anwendung statistischer Verfahren genauer oder durch eine Vergrößerung der Anzahl der enthaltenen Stützpunkte dichter. Die Quantität der solcherart zu gewinnenden Informationen ist nicht durch das Verfahren oder die Vorrichtung selbst, sondern lediglich durch die Größe des zur Verfügung stehenden Speichers der Datenverarbeitungseinheit begrenzt.

Die Verwendung der oben beschriebenen Strahlquellen für kurze Strahlimpulse ist im vorstehend beschriebenen Zusammmenhang erfindungsgemäß besonders vorteilhaft einzusetzen, um die Verwackelungseffekte einer beispielsweise von Hand geführten Aufnahmevorrichtung zu minimieren.

Die erfindungsgemäß vorteilhafte Verwendung mindestens einer Strahlquelle, die mittels geeigneter optischer Mittel ein Muster unter einem ausreichenden Triangulationswinkel zur Aufnahmerichtung auf die zu erfassende Oberfläche projiziert und mindestens einer weiteren Strahlquelle, die das Objekt beispielsweise alternierend farbgetreu beleuchtet, sowie die anschließende Kombination der den Einzelbildern zugehörigen 3D-Informationen mit den den Einzelbildern zugeordneten Farbinformationen erlaubt eine äußerst realitätsnahe Einfärbung einer resultierenden 3D-Gitternetzdarstellung.

Im vorstehenden Zusammenhang ist es erfindungsgemäß besonderes vorteilhaft, diejenige Strahlquelle, die beispielsweise mittels Kondensor, Maske und Optik ein Linienmuster auf die zu erfassende Oberfläche projiziert, mit hoher Strahlintensität zu betreiben, damit für die Gewinnung der 3D-Information das projizierte Muster in allen Bereichen der Oberfläche hinreichend hell ist. Dabei ergibt sich, daß diejenigen Bereiche der Oberfläche mit einer starken Reflexion unzureichende Kontrastund/oder Farbinformationen zum Zweck einer anschaulichen Darstellung beinhalten. Die fehlenden Informationen können wie oben beschrieben durch Kombination von Einzelbildinformationen ergänzt werden, die mit reduzierter Bestrahlung beispielsweise aus derselben Strahlquelle oder vermittels der ebenfalls beschriebenen Strahlquelle für die flächige Bestrahlung der zu erfassenden Oberfläche gewonnen werden.

Es ist im vorstehenden Zusammenhang erfindungsgemäß besonders vorteilhaft, die Strahlenergie für die Projektion des Musters für die Einzelaufnahmen unterschiedlich zu stellen, um auch bei der Aufnahme des Linienmusters - wie bereits oben für die flächige Bildaufnahme beschrieben- die Bildinformationen des Linienmusters bezogen auf die partiellen Reflexionseigenschaften der Oberfläche des Objekts durch eine Kombination der Einzelaufnahmen insgesamt zu optimieren.

Es ist in .diesem Zusammenhang erfindungsgemäß besonders vorteilhaft, einen Farbbildwandler und für die Projektion des Linienmusters weißes Licht zu verwenden. Variiert man nun beispielsweise unter Verwendung einer Blitzlampe die Strahlenergie wie vorstehend beschrieben, erhält man in der Folge der Einzelbilder sowohl optimale Farb- und Kontrastinformationen entlang der projizierten Linien als auch optimale Kontrastsignale für die einzelnen Linien zur Berechnung der 3D-Stützpunkte. Bewegt man nun mit geringer Geschwindigkeit die Aufnahmevorrichtung gegenüber dem Objekt, kann durch geeignete Kombination der den Einzelbildem zugeordneten Informationen sowohl die 3D-Informationen verdichtet als auch ein Farbbild des Objekts ohne Lücken generiert werden. In diesem Sinne kann die vorstehend noch beschriebene einheitlich flächige Beleuchtung des Objekts entfallen.

Die Vorrichtung zur Durchführung von optischen Aufnahmen der eingangs beschriebenen Art löst die Aufgabe gemäß der Erfindung durch die Merkmale, daß
a) mindestens ein flächiger elektronischer Bildwandler unter Verwendung einer zur Aufnahme von Bildern geeigneten Optik verwendet wird, der sich für die Aufnahme von mindestens zwei aufeinanderfolgenden Bildern eignet und
b) mindestens ein stellbares optisches Mittel vorgesehen wird, das sich für die Aufnahme der Einzelbilder hinsichtlich der für die Bildwandlung wirksam eingebrachten Menge der Strahlenergie unterschiedlich stellen läßt.

Eine erfindungsgemäß besonders vorteilhafte Ausbildung der Vorrichtung sieht Einrichtungen vor, die die Digitalisierung der Ausgangssignale des mindestens einen Bildwandlers bewirken und diese Daten einer Datenverarbeitungsanlage zur Verfügung stellt.

Eine erfindungsgemäß besonders vorteilhafte Ausbildung der Vorrichtung sieht Einrichtungen vor, die die Daten von mindestens zwei Einzelbildern speichem können.

Eine erfindungsgemäß besonders vorteilhafte Ausbildung der Vorrichtung sieht Einrichtungen vor, die die Daten von mindestens zwei Einzelbildern verarbeitet und in der Lage ist, diese mittels geeigneter Algorithmen zu kombinieren.

Eine erfindungsgemäß besonders vorteilhafte Ausbildung der Vorrichtung sieht optische Mittel im Strahlengang zwischen der mindestens einen Strahlquelle und dem zu erfassenden Objekt vor, die die Projektion eines Musters erlauben.

Weitere Vorteile, Merkmale und Anwendungsmöglichkeiten der vorliegenden Erfindung ergeben sich aus der folgenden Beschreibung bevorzugter Ausführungsbeispiele in Verbindung mit der anliegenden Zeichnung.

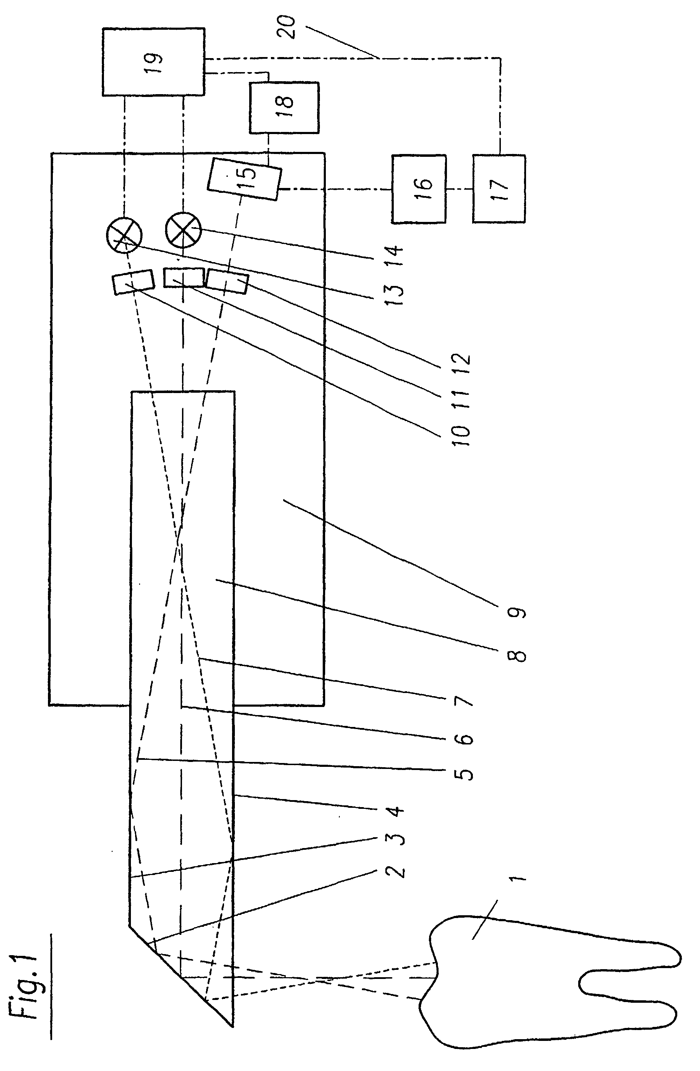

Es zeigt die anliegende Zeichnung (Fig. 1) die Ansicht einer Vorrichtung zur Durchführung von optischen Aufnahmen nach der Erfindung, teilweise schematisiert.

Die Aufnahmevorrichtung gemäß der Fig. 1 ist zur Vereinfachung der Beschreibung ohne bekannte, selbstverständliche Vorrichtungsteile nur schematisch wiedergegeben. Zur Vereinfachung der Beschreibung wurde zudem darauf verzichtet, Ausführungsformen von Vorrichtungsteilen, die zum Stand der Technik gehören, wie bestimmte Ausführungsformen von Verbindungen oder der Fixierung von Teilen, im Detail zu beschreiben.

An einem Grundgestell (9) sind alle Mittel (8 und 10 bis 15) starr befestigt. Die Befestigung des Trägers (8) gegenüber dem Grundgestell (9) ist lösbar und in der hier gezeigten Ausführungsform derart ausgebildet, daß sich bei einem erneuten Zusammenbau die vorgesehene geometrische Anordnung dieser Teile zueinander ohne Justagearbeiten ergibt. Zudem sind der Träger (8) und die mit diesem starr verbundenen optischen Mittel (2 bis 4) in der hier gezeigten Ausführungsform derart ausgebildet, daß sie sich für eine von den übrigen Mitteln (9 bis 20) separate Desinfektion bzw. Sterilisation eignen.

Bei dem Objekt (1) handelt es sich um eine Oberfläche mit dreidimensionalen Erstreckungen. Beispielsweise kann es sich um einen Zahn bzw. um eine Reihe von Zähnen des menschlichen Gebisses handeln. In engem Abstand erstreckt sich der Träger (8). mit seinen Aufbauten, und man kann sich leicht vorstellen, daß der in Richtung Objekt (1) weisende Teil des Trägers (8) mit seinen Aufbauten innerhalb der Mundhöhle untergebracht werden kann. Es ist weiter leicht vorstellbar, daß der aus den Mitteln (2 bis 15) bestehende Teil der Aufnahmevorrichtung von Hand gehalten wird und der Benutzer der Vorrichtung einen intraoralen Aufnahmevorgang von einem Teil des Gebjsses eines Patienten, in Fig. 1 gezeigt als Objekt (1), ausführt.

In der hier gezeigten Ausführungsform handelt es sich bei dem Träger (8) um einen prismatischen Körper aus optischem Glas der Güte BK 7 mit einem Querschnitt von 15 mm x 15 mm. Die Längsseiten sind parallel geschliffen, poliert und eignen sich in dieser Ausführung als ebene optische Umlenkeinrichtungen unter Ausnutzung der Totalreflexion, in Fig. 1 gezeigt als Spiegel (3 und 4). Der zum Objekt (1) weisende Teil des Glasprismas, in Fig. 1 gezeigt als Träger (8), ist unter 45° abgeschrägt. Diese Fläche ist ebenfalls geschliffen, poliert und zudem verspiegelt und dient als ebener Spiegel (2) zur Umlenkung der Strahlen in Richtung des Objekts (1).

Aus Platzgründen kann es vorteilhaft sein, auf der vom Objekt (1) abweisenden Seite des Trägers (8) ebenfalls Umlenkeinheiten anzuordnen, damit die Mittel (10 bis 15) nicht in einer Ebene angeordnet werden müssen.

Spiegel sind **im Sinne dieser Erfindung** optische Umlenkeinrichtungen unabhängig davon, ob die relevanten Flächen verspiegelt sind oder die Fähigkeit zur Umlenkung optischer Strahlen unter Ausnutzung der Totalreflexion erfolgt.

Der als Glasprisma ausgeführte Träger (8) kann erfindungsgemäß vorteilhaft beheizt werden, damit er bei invasiv durchgeführten Aufnahmen nicht beschlägt. Alternativ kann ein Luftstrom derart geführt werden, daß der intraoral eingesetzte und für die Aufnahme relevante Teil des Glaskörpers trocken gehalten wird.

In einer anderen vorteilhaften Ausgestaltung der Vorichtung wird der Träger (8) derart ausgebildet, daß er sich für endoskopische Untersuchungen eignet. Zudem ist es, erfindungsgemäß vorteilhaft, möglich weitere optische Mittel, beispielsweise Linsen, zusätzlich zum bzw. anstelle des Spiegels (2) auf dem Träger (8) anzuordnen.

Die Strahlquellen (13 und 14), jeweils versehen mit den optischen Mitteln (10 und 11), dienen zur Beleuchtung des Objekts (1). Die Strahlen werden über die ebenen Spiegel (2 bis 4) umgelenkt. Bei der hier gezeigten Ausführungsform bestehen die optischen Mittel (10) aus einem Kondensor, einer Maske und einem Objektiv. Vermittels der Mittel (13, 10, 4, 2) wird flächig ein Linienmuster auf das Objekt (1) projiziert. Als optisches Mittel (11) findet ein Kondensor Verwendung. Vermittels der Mittel (14, 11 und 2) läßt sich das Objekt flächig beleuchten. Als Strahlquelle (13 und 14) kommen Blitzlampen zum Einsatz. Die Strahlquellen (13 und 14) sind vermittels elektrischer Verbindungen (20) mit einer Ansteuereinheit (19) verbunden.

Die vom Objekt (1) reflektierten Strahlen werden über die Spiegel (2 und 3) gelenkt und vermittels der optischen Mittel (12) auf dem Bildwandler (15) abgebildet. Bei der hier gezeigten Ausführungsform bestehen die optischen Mittel (12) aus einem Objektiv. Der elektronische Bildwandler (15) ist als CCD-Array ausgebildet und über elektrische Verbindungen (20) mit einer Ansteuereinrichtung (18) und einer Einrichtung zur Digitalisierung des Ausgangssignals des Bildwandlers (16) verbunden. Die digitalisierten Bilddaten werden über die elektische Verbindung (20) einer Datenverarbeitungseinheit (17) zur Verfügung gestellt. In der hier gezeigten Ausführungsform wird für die Einheit (16) ein sogenannter Frame-Grabber mit eigenem Signalprozessor für die schnelle Verarbeitung der Bilddaten und für die Einheit (17) ein handelsüblicher PC verwendet.

Die elektrischen Verbindungen (20) zwischen den Einrichtungen (18) und (19) sowie zwischen den Einrichtungen (17) und (19) dienen zur Synchronisation der Ansteuerung der Strahlquellen (13 und 14) mit der Bildwechselfrequenz des Bildwandlers (15) bzw. zur Rückführung der Bildinformation in einem Regelkreis zur Optimierung der Ansteuerung der Strahlquellen.

Die Mittel (13, 10 und 4) einerseits und (15, 12 und 3) andererseits sind bezüglich ihrer optischen Achsen (7 und 5) derart ausgerichtet, daß sich zwischen der Projektionsrichtung des Musters und der Richtung der Bildaufnahme ein Triangulationswinkel von 20 Grad ergibt. Die optische Achse (6) für die flächige Beleuchtung liegt in diesem Ausführungsbeispiel in der Winkelhalbierenden der beiden anderen optischen Achsen (5 und 7). Um für die 3D-Berechnung relevante Informationen zu bekommen, ist das projizierte Linienmuster quer zu der von dem Triangulationswinkel aufgespannten Ebene gestreift.

In der hier gezeigten Ausführungsform wird das Objekt (1) synchron zur Bildwechselfrequenz des Bildwandlers (15) alternierend einerseits mit einem Linienmuster und andererseits flächig/einheitlich beleuchtet. Die Bildwechselfrequenz liegt in dem hier gezeigten Ausführungsbeispiel bei 50 Hz.

Für die optische dreidimensionale Erfassung beispielsweise des Zahnbogens eines Menschen führt man den vorderen Teil des Trägers (8) mit seinen Aufbauten in den Mund des Patienten ein, startet den Aufnahmevorgang durch ein in der Fig. 1 nicht gezeigtes, im Rahmen der Datenverarbeitungseinheit (17) verfügbares Betätigungselement, und führt im Verlauf des Aufnahmevorgangs den Vorrichtungsteil, bestehend aus den Mitteln (2 bis 15) von Hand derart, daß nach und nach alle relevanten Oberflächenbereiche des Objekts (1) sowohl auf dem Bildwandler (15) abgebildet als auch gleichermaßen von der Projektion des Linienmusters erfaßt werden. Man beendet den Aufnahmevorgang durch eine weitere Betätigung des vorstehend genannten Betätigungselements.

In der Folge der aufgenommenen Einzelbildinformationen befinden sich jetzt abwechselnd Abbilder des aufgrund der Oberflächengestalt des Objekts (1) verzeichneten Linienmusters und Abbilder des flächig beleuchteten Objekts (1). Aus der Verzeichnung des Linienmusters lassen sich bei Kenntnis des optischen Strahlengangs und unter Berücksichtigung der geometrischen Ausbildung des projizierten Linienmusters für die entsprechenden Einzelaufnahmen für eine Vielzahl von Stützpunkten 3D-Koordinaten errechnen. Aus der Folge der den Einzelbildern zugeordneten Bildinformationen und 3D-Koordinaten lassen sich im weiteren sowohl die ebenen Bildinformationen als auch die 3D-Koordinaten in der oben beschriebenen Art und Weise kombinieren, so daß im Ergebnis trotz des Sichtfelds der Aufnahmeeinheit in einer Größenordnung von 15 mm x 15 mm sowohl die 3D-Koordinaten als auch ein einheitliches Farbbild des gesamten Zahnbogens vorliegen. Hierbei sind Lücken aufgrund von Hinterschneidungen, störenden optischen Reflexionen oder dunklen Bildbereichen ohne nennenswerten Kontrast nicht mehr vorhanden. Durch eine online-Berechnung und -Anzeige der jeweiligen Zwischenergebnisse kann der Bediener zudem die Führung der Aufnahmevorrichtung von Hand optimieren.

Die für die 3D-Berechnung erforderliche Kenntnis des optischen Strahlengangs und der geometrischen Ausbildung des projizierten Linienmusters erhält man erfindungsgemäß vorteilhaft dadurch, daß man nacheinander in unterschiedlichem Abstand parallel zu der zum Objekt (1) weisenden Fläche des Trägers (8) ein ebenes, in seiner Detailausbildung in der Fig. 1 nicht gezeigtes Objekt (1) positioniert, das quer zum projizierten Linienmuster ebenfalls ein Linienmuster aufweist. Werden nun zwei Bildaufnahmen angefertigt und ist sowohl das Referenz-Objekt (1) eben und das Linienmuster in seiner geometrischen Ausbildung als auch der Abstand zwischen Träger und Referenz-Objekt (8) für beide Aufnahmen bekannt, kann man für eine Vielzahl von Stützpunkten sowohl den Strahlengang als auch die geometrische Ausbildung des projizierten Musters exakt errechnen. Die errechneten Werte berücksichtigen bereits etwaige Verzeichnungen aufgrund der Fertigungstoleranzen der verwendeten Mittel. Die Zwischenwerte können dann im weiteren bei der 3D-Berechnung interpoliert werden.

Die hier gezeigte Ausführungsform zeichnet sich dadurch aus, daß
a) in der Aufnahmeeinheit keine bewegten Teile Verwendung finden;
b) der optische Aufbau einfach ausgebildet ist;
c) durch die Verwendung eines langgestreckten Glaskörpers eine einfache Möglichkeit gewählt wurde, die Strahlen zur Erzeugung eines Musters auf der zu vermessenden Oberfläche gezielt ein- oder mehrfach an den Wänden des Glaskörpers reflektieren zu lassen, so daß sich eine größere Winkeldifferenz relativ zu den Aufnahmestrahlen ergibt, als wenn alle Strahlen geradlinig durch den Träger geführt werden, wobei sich der relative Winkel der Strahlen zueinander ja lediglich als arcus tangens des Verhältnisses vom Abstand zwischen Objekt und Aufnahme- bzw. Beleuchtungseinheiten einerseits und dem. Abstand der Optiken untereinander andererseits ergibt. Eine Winkeldifferenz im Bereich zwischen 15° und 45° zwischen den mustererzeugenden Strahlen und den Strahlen für die Aufnahme ist vorteilhaft für die Durchführung der Vermessung. Größere Winkel vergrößern die Gefahr der "Schattenbildung" bei gestuften Oberflächen, kleinere Winkel vermindern die Genauigkeit der Vermessung, da das Auswertungsverfahren auf Triangulationsberechnungen beruht;
d) sich außer für Träger (8), Grundgestell (9), Maske zur Projektion des Musters und Ansteuereinheiten (18 und 19) handelsübliche Hardware-Komponenten verwenden lassen;
e) sich die Fehler aufgrund der Fertigungstoleranzen der eingesetzten Mittel durch die oben beschriebene "Kalibrierung" der Aufnahmeeinheit kompensieren lassen und insofern insgesamt geringere Anforderungen an die Fertigungstoleranzen der eingesetzten Mittel gestellt werden müssen.

### Bezugszeichenliste

- 1: Objekt
- 2, 3, 4: Spiegel
- 5, 6, 7: optische Achsen
- 8: Träger (hier ausgeführt als Körper aus optischem Glas)
- 9: Grundgestell
- 10, 11, 12: optische Mittel (Linsensystem, Blende etc.)
- 13, 14: Strahlquelle (hier ausgeführt als Blitzlampe)
- 15: elektronischer Bildwandler (CCD-Array)
- 16: Einrichtung zur Digitalisierung analoger Signale (Frame-Grabber)
- 17: Datenverarbeitungseinheit (PC)
- 18: Ansteuereinheit für den Bildwandler
- 19: Ansteuereinheit für die Strahlquellen
- 20: elektrische Verbindungen

## Patentansprüche

1. Verfahren zur Durchführung von optischen Aufnahmen zum Zweck der Darstellung, Dokumentation oder Vermessung von Objekten (1) unter Verwendung mindestens eines elektronischen Bildwandlers (15) mit flächigem Sichtfeld, mindestens eines optischen Mittels (12) für die Abbildung des Objektes auf dem Bildwandler (15) und mindestens einer Strahlquelle (13, 14) für die Bestrahlung bzw. Durchstrahlung des Objekts (1), **dadurch gekennzeichnet, daß**
a) mindestens zwei Einzelaufnahmen nacheinander erfolgen und
b) die wirksame Menge der eingebrachten Strahlenergie für die optoelektronische Wandlung der Bilder für die Aufnahme der Einzelbilder unterschiedlich gestellt wird.

2. Verfahren nach Anspruch 1, **dadurch gekennzeichnet, daß** die Intensität der eingebrachten Strahlenergie für die optoelektronische Wandlung der Bilder für die Aufnahme der Einzelbilder unterschiedlich gestellt wird.

3. Verfahren nach Anspruch 1 oder 2, **dadurch gekennzeichnet, daß** die Wirkungsdauer der eingebrachten Strahlenergie für die optoelektronische Wandlung der Bilder für die Aufnahme der Einzelbilder unterschiedlich gestellt wird.

4. Verfahren nach einem der Ansprüche 1 bis 3, **dadurch gekennzeichnet, daß** mindestens eine Strahlquelle (13, 14) bezüglich der von ihr für die Aufnahme eines Einzelbildes abgegebenen Menge an Strahlenergie im Verlauf der Aufnahme der Einzelbilder unterschiedlich gestellt wird.

5. Verfahren nach einem der Ansprüche 1 bis 4, **dadurch gekennzeichnet, daß** optische Mittel (10, 11) für die Beeinflussung der für die Aufnahme eines Einzelbildes wirksamen Menge an Strahlenergie in mindestens einem Strahlengang (6, 7) zwischen der mindestens einen Strahlquelle (13, 14) und dem zu erfassenden Objekt (1) im Verlauf der Aufnahme der Einzelbilder unterschiedlich gestellt werden.

6. Verfahren nach einem der Ansprüche 1 bis 5, **dadurch gekennzeichnet, daß** optische Mittel (12) für die Beeinflussung der für die Aufnahme eines Einzelbildes wirksamen Menge an Strahlenergie in mindestens einem Strahlengang (5) zwischen dem zu erfassenden Objekt (1) und dem mindestens einen Bildwandler (15) im Verlauf der Aufnahme der Einzelbilder unterschiedlich gestellt werden.

7. Verfahren nach einem der Ansprüche 1 bis 6, **dadurch gekennzeichnet, daß** sogenannte Shutter-Einrichtungen des mindestens einen elektronischen Bildwandlers (15) mit Hilfe geeigneter Mittel (18) derart angesteuert werden, daß die Wirkungsdauer der eingebrachten Strahlenergie für die optoelektronische Wandlung der Bilder im Verlauf der Aufnahme der Einzelbilder unterschiedlich gestellt wird.

8. Verfahren nach einem der Ansprüche 1 bis 7, **dadurch gekennzeichnet, daß** die Ausgangssignale des mindestens einen Bildwandlers (15) in einer entsprechenden Einheit (16) digitalisiert werden und die digitalisierten Ausgangssignale einer Datenverarbeitungsanlage (17) zur Verfügung gestellt werden.

9. Verfahren nach Anspruch 8, **dadurch gekennzeichnet, daß** Bilddaten von mindestens zwei Einzelbildem in einer Datenverarbeitungsanlage (17) gespeichert, dargestellt und/oder mittels geeigneter Algorithmen kombiniert werden.

10. Verfahren nach einem der Ansprüche 1 bis 9, **dadurch gekennzeichnet, daß** die für die optoelektronische Wandlung der Bilder wirksame Menge an Strahlenergie synchron zur Bildwechselfrequenz des Bildwandlers (15) unterschiedlich gestellt wird.

11. Verfahren nach einem der Ansprüche 1 bis 10, **dadurch gekennzeichnet, daß** die für die optoelektronische Wandlung der Bilder wirksame Menge an Strahlenergie in einer Steuerstrecke unterschiedlich gestellt wird.

12. Verfahren nach einem der Ansprüche 1 bis 11, **dadurch gekennzeichnet, daß** die für die optoelektronische Wandlung der Bilder wirksame Menge an Strahlenergie in einem Regelkreis unterschiedlich gestellt wird.

13. Verfahren nach einem der Ansprüche 1 bis 12, **dadurch gekennzeichnet, daß** optische Mittel (10) in mindestens einem Strahlengang zwischen der mindestens einen Strahlquelle (13, 14) und dem zu erfassenden Objekt (1) vorgesehen werden, die die Projektion mindestens eines Musters erlauben.

14. Verfahren nach einem der Ansprüche 1 bis 13, **dadurch gekennzeichnet, daß** in der Datenverarbeitungseinheit (17) Einrichtungen und/oder Algorithmen zur Bildung einer 3D-Information verwendet werden.

15. Verwendung der Verfahren nach einem der Ansprüche 1 bis 14 für die medizinische Diagnostik oder Therapie.

16. Vorrichtung für ein Verfahren nach einem der Ansprüche 1 bis 14, **dadurch gekennzeichnet, daß** mindestens ein Bildwandler (15) als CCD-Array ausgebildet ist.

17. Vorrichtung für ein Verfahren nach einem der Ansprüche 1 bis 14, **dadurch gekennzeichnet, daß** mindestens eine Strahlquelle (13, 14) als Blitzstrahlkörper ausgebildet ist.

18. Vorrichtung für ein Verfahren nach einem der Ansprüche 1 bis 14, **dadurch gekennzeichnet, daß** mindestens ein Grundgestell (9) oder ein Träger (8) mindestens zwei der Mittel (2 bis 4 und 10 bis 15) starr verbindet.

19. Vorrichtung nach Anspruch 18, **dadurch gekennzeichnet, daß** an dem Träger (8) mindestens ein Linsensystem (12) zur optischen Abbildung. des Objekts (1) auf dem Bildwandler (15) befestigt ist.

20. Vorrichtung nach Anspruch 18, **dadurch gekennzeichnet, daß** an dem Träger (8) mindestens eine optische Umlenkeinrichtung (2, 3, 4) befestigt ist.

21. Vorrichtung nach einem der Ansprüche 18 bis 20, **dadurch gekennzeichnet, daß** der Träger (8) mit seinen Aufbauten gegenüber den anderen Mitteln (9 bis 20) lösbar und im übrigen derart ausgebildet ist, daß er sich für eine separate Sterilisation bzw. Desinfektion eignet.

22. Verwendung der Vorrichtungen nach einem der Ansprüche 16 bis 21 für invasive medizinische Zwecke.

23. Verwendung der Vorrichtungen nach einem der Ansprüche 16 bis 21 zur berührungslosen Vermessung von wenigstens einem Zahn eines Gebisses des menschlichen und tierischen Körpers für zahnmedizinische Zwecke.

24. Verwendung der Vorrichtungen nach einem der Ansprüche 16 bis 21 zur berührungslosen Vermessung des Zahnbogens eines Gebisses des menschlichen und tierischen Körpers für kieferorthopädische Zwecke.

## Claims

1. Method for carrying out optical pick up for the purpose of representation, documentation or surveying of objects (1) using at least one electronic image converter (15) with an areal viewing field, at least one optical means (12) for imaging the object on the image converter (15) and at least one beam source (13, 14) for illuminating or transilluminating the object (1), **characterised in that**
a) at least two single images follow one another consecutively, and
b) the effective amount of beam energy applied for the optoelectronic conversion of the images is set differently for picking up the single images.

2. Method according to claim 1, **characterised in that** the intensity of the beam energy applied for the optoelectronic conversion of the images is set differently for picking up the single images.

3. Method according to claim 1 or 2, **characterised in that** the duration of action of the beam energy applied for the optoelectronic conversion of the images is set differently for picking up the single images.

4. Method according to one of claims 1 to 3, **characterised in that** at least one beam source (13, 14) is set differently with respect to the amount of beam energy it emits for picking up a single image during picking up of the single images.

5. Method according to one of claims 1 to 4, **characterised in that** optical means (10, 11) for influencing the effective amount of beam energy for picking up of a single image in at least one beam path (6, 7) between the at least one beam source (13, 14) and the object (1) to be determined are set differently during picking up of the single images.

6. Method according to one of claims 1 to 5, **characterised in that** optical means (12) for influencing the effective amount of beam energy for picking up a single image in at least one beam path (5) between the object (1) to be determined and the at least one image converter (15) is set differently during picking up of the single images.

7. Method according to one of claims 1 to 6, **characterised in that** the so-called shutter means of the at least one electronic image converter (15) are controlled, with the aid of suitable means (18), such that the duration of action of the beam energy applied for the optoelectronic conversion of the images is set differently during picking up of the single images.

8. Method according to one of claims 1 to 7, **characterised in that** the output signals of the at least one image converter (15) are digitised in a suitable unit (16), and the digitised output signals are made available to a data processing system (17).

9. Method according to claim 8, **characterised in that** image data from at least two single images are stored, represented and/or combined by means of suitable algorithms, in a data processing system (17).

10. Method according to one of claims 1 to 9, **characterised in that** the amount of beam energy effective for optoelectronic conversion of the images is set differently, synchronously with the image refresh rate of the image converter (15).

11. Method according to one of claims 1 to 10, **characterised in that** the amount of beam energy effective for optoelectronic conversion of the images is set differently in a control section.

12. Method according to one of claims 1 to 11, **characterised in that** the amount of beam energy effective for optoelectronic conversion of the images is set differently in a control loop.

13. Method according to one of claims 1 to 12, **characterised in that** optical means (10) are provided in at least one beam path between the at least one beam source (13, 14) and the object (1) to be determined, which allow the projection of at least one pattern.

14. Method according to one of claims 1 to 13, **characterised in that** means and/or algorithms for forming 3D data are used in the data processing unit (17).

15. Use of the method according to one of claims 1 to 14, for medical diagnostics or therapy.

16. Device for a method according to one of claims 1 to 14, **characterised in that** at least one image converter (15) is in the form of a CCD array.

17. Device for a method according to one of claims 1 to 14, **characterised in that** at least one beam source (13, 14) is in the form of a flash body.

18. Device for a method according to one of claims 1 to 14, **characterised in that** at least one base frame (9) or a carrier (8) rigidly connects at least two of the means (2 to 4 and 10 to 15).

19. Device according to claim 18, **characterised in that** at least one lens system (12) for optical imaging of the object (1) on the image converter (15) is fixed to the carrier (8).

20. Device according to claim 18, **characterised in that** at least one optical deflector means (2, 3, 4) is fixed to the carrier (8).

21. Device according to one of claims 18 to 20, **characterised in that** the carrier (8) together with its components is releasable with respect to the other means (9 to 20) and is moreover of such a configuration that it is suitable for separate sterilisation and disinfection.

22. Use of the devices according to one of claims 16 to 21 for invasive medical purposes.

23. Use of the devices according to one of claims 16 to 21 for non-contact surveying of at least one tooth of a set of teeth of the human or animal body, for dentistry purposes.

24. Use of the devices according to one of claims 16 to 21 for non-contact surveying of the tooth set of a set of teeth of the human or animal body, for orthodontic purposes.

## Revendications

1. Procédé d'enregistrements optiques destiné à la représentation, la documentation ou la topographie d'objets (1) en utilisant au moins un convertisseur d'image (15) à champ visuel plan, au moins un moyen optique (12) destiné à représenter l'objet sur le convertisseur d'image (15) et au moins une source de rayonnement (13, 14) destinée à irradier ou radiographier l'objet (1), **caractérisé en ce que**
a) au moins deux enregistrements individuels sont effectués successivement et
b) la quantité efficace d'énergie de rayonnement apportée pour la conversion optoélectronique des images est réglée de façon variable en vue d'enregistrer des images individuelles.

2. Procédé selon la revendication 1, **caractérisé en ce que** l'intensité de l'énergie de rayonnement apportée pour la conversion optoélectronique des images est réglée de façon variable en vue d'enregistrer des images individuelles.

3. Procédé selon la revendication 1 ou 2, **caractérisé en ce que** la durée efficace de l'énergie de rayonnement apportée pour la conversion des images est réglée de façon variable en vue d'enregistrer des images individuelles.

4. Procédé selon l'une des revendications 1 à 3, **caractérisé en ce que** au moins une source de rayonnement (13, 14) est réglée de façon variable, quant à la quantité d'énergie de rayonnement que cette source délivre pour l'enregistrement d'une image individuelle, au cours de l'enregistrement des images individuelles.

5. Procédé selon l'une des revendications 1 à 4, **caractérisé en ce que** des moyens optiques (10, 11) destinés à influer sur la quantité d'énergie de rayonnement efficace pour enregistrer une image individuelle est fournie de façon variable sur au moins un trajet de rayon (6, 7) entre l'au moins une source de rayonnement (13, 14) et l'objet à enregistrer (1) au cours de l'enregistrement des images individuelles.

6. Procédé selon l'une des revendications 1 à 5, **caractérisé en ce que** des moyens optiques (12) destinés à influer sur la quantité d'énergie de rayonnement efficace pour enregistrer une image individuelle est réglée de façon variable sur au moins un trajet de rayon (5) entre l'objet à enregistrer
(1) et l'au moins un convertisseur d'image (15) au cours de l'enregistrement des images individuelles.

7. Procédé selon l'une des revendications 1 à 6, **caractérisé en ce que** des dispositifs dits à obturateurs de l'au moins un convertisseur d'image électronique (15) sont commandés à l'aide de moyens appropriés (18) de façon à régler de façon variable la durée d'action de l'énergie de rayonnement apportée pour la conversion optoélectronique des images au cours de l'enregistrement des images individuelles.

8. Procédé selon l'une des revendications 1 à 7, **caractérisé en ce que** les signaux de sortie de l'au moins un convertisseur d'image (15) sont numérisées dans une unité appropriée (18) et les signaux de sortie numérisés sont envoyés à une installation de traitement de données (17).

9. Procédé selon la revendication 8, **caractérisé en ce que** des données d'image d'au moins deux images individuelles sont mémorisées, visualisées et/ou combinées au moyen d'algorithmes appropriés dans une installation de traitement de données (17).

10. Procédé selon l'une des revendications 1 à 9, **caractérisé en ce que** la quantité efficace d'énergie de rayonnement pour la conversion optoélectronique des images est réglée de façon variable en étant synchronisée sur la fréquence d'image du convertisseur d'image (15).

11. Procédé selon l'une des revendications 1 à 10, **caractérisé en ce que** la quantité efficace d'énergie de rayonnement pour la conversion optoélectronique des images est réglée de façon variable dans une plage de commande.

12. Procédé selon l'une des revendications 1 à 11, **caractérisé en ce que** la quantité efficace d'énergie de rayonnement pour la conversion optoélectronique des images est réglée de façon variable dans un circuit de régulation.

13. Procédé selon l'une des revendications 1 à 12, **caractérisé en ce que** des moyens optiques (10) sont prévus sur au moins un trajet de rayon entre l'au moins une source de rayonnement (13, 14) et l'objet à enregistrer (1), lesquels moyens optiques permettent la projection d'au moins un échantillon.

14. Procédé selon l'une des revendications 1 à 13, **caractérisé en ce que** des dispositifs et/ou des algorithmes sont utilisés pour former une information 3D dans l'unité de traitement données (17).

15. Utilisation du procédé selon l'une des revendications 1 à 14 en médecine pour le diagnostic ou la thérapie.

16. Appareil de mise en oeuvre d'un procédé selon l'une des revendications 1 à 14, **caractérisé en ce que** au moins un convertisseur d'image (15) se présente sous la forme d'un réseau CCD.

17. Appareil de mise en oeuvre d'un procédé selon l'une des revendications 1 à 14, **caractérisé en ce que** au moins une source de rayonnement (13, 14) se présente sous la forme d'un corps rayonnant par flashes.

18. Appareil de mise en oeuvre d'un procédé selon l'une des revendications 1 à 14, **caractérisé en ce que** au moins un châssis (9) ou un support (8) relie rigidement au moins deux des moyens (2 à 4 et 10 à 15).

19. Appareil selon la revendication 18, **caractérisé en ce que** au moins un système de lentilles (12) est fixé au support (8) pour représenter optiquement l'objet (1) sur le convertisseur d'image (15).

20. Appareil selon la revendication 18, **caractérisé en ce que** au moins un dispositif de déviation optique (2, 3, 4) est fixé au support (8).

21. Appareil selon l'une des revendications 18 à 20, **caractérisé en ce que** le support (8) est démontable avec son carter des autres moyens (9 à 20) et est conformé par ailleurs de façon à être approprié à une stérilisation ou désinfection séparée.

22. Utilisation des appareils selon l'une des revendications 16 à 21 en médecine exploratrice.

23. Utilisation des appareils selon l'une des revendications 16 à 21 pour la topographie sans contact d'au moins une dent d'une denture du corps humain et animal en odontologie.

24. Utilisation des appareils selon l'une des revendications 16 à 21 pour la topographie sans contact de la mâchoire d'une denture du corps humain ou animal en odontologie orthopédique.
